(19) Europäisches Patentamt
European Patent Office
Office européen des brevets

(11) Veröffentlichungsnummer: **0 543 263 A2**

# (12) EUROPÄISCHE PATENTANMELDUNG

(21) Anmeldenummer: **92119261.3**

(22) Anmeldetag: **11.11.92**

(51) Int. Cl.5: **C07D 235/14**, A61K 31/415, C07D 235/08, C07D 403/04, C07D 403/14, C07D 401/04, C07D 471/04, C07D 401/14

(30) Priorität: **16.11.91 DE 4137812**
**30.01.92 YU 98/92**

(43) Veröffentlichungstag der Anmeldung:
**26.05.93 Patentblatt 93/21**

(84) Benannte Vertragsstaaten:
**ES**

(71) Anmelder: **Dr. Karl Thomae GmbH**
**Postfach 1755**
**W-7950 Biberach 1(DE)**

(72) Erfinder: **Hauel, Norbert, Dr. Dipl.-Chem.**
**Marderweg 12**
**W-7957 Schemmerhofen(DE)**
Erfinder: **Narr, Berthold, Dr. Dipl.-Chem.**
**Obere Au 5**
**W-7950 Biberach 1(DE)**
Erfinder: **Riess, Uwe, Dr. Dipl.-Chem.**
**Dunantstrasse 10**
**W-7950 Biberach 1(DE)**
Erfinder: **Meel, Jacques van, Dr. Pharm.**
**Schubertweg 4**
**W-7951 Mittelbiberach(DE)**
Erfinder: **Wienen, Wolfgang, Dr. Dipl.-Biol.**
**Am Schiessberg 28**
**W-7951 Äpfingen(DE)**
Erfinder: **Entzeroth, Michael, Dr. Dipl.-Chem.**
**Sebastian-Sailer-Strasse 44**
**W-7951 Warthausen(DE)**

(54) **Benzimidazole, diese Verbindungen enthaltende Arzneimittel und Verfahren zu ihrer Herstellung.**

(57) Die Erfindung betrifft Benzimidazole der allgemeinen Formel

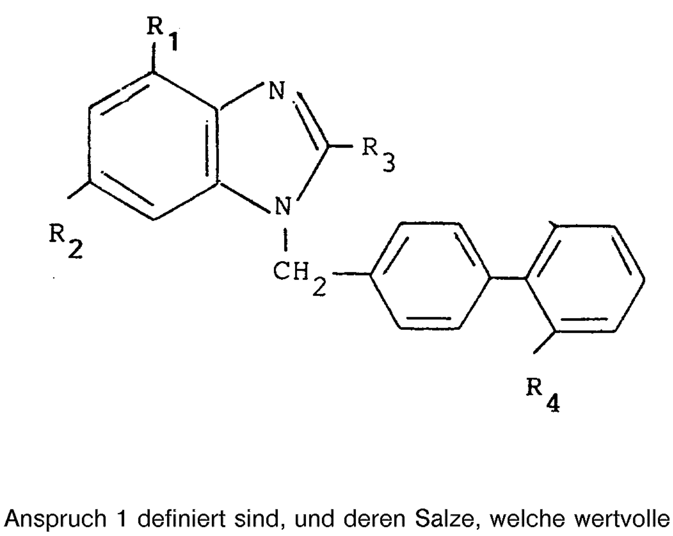

,(I)

in der
$R_1$ bis $R_4$ wie im Anspruch 1 definiert sind, und deren Salze, welche wertvolle Eigenschaften aufweisen.
Die neuen Verbindungen stellen insbesondere Angiotensin-Antagonisten dar.

EP 0 543 263 A2

In der US-A-4,880,804 werden u.a. 4'-[(2-Alkyl-benzimidazol-1-yl)-methyl]biphenyl-2-carbonsäuren und 4'-[(2-Alkyl-benzimidazol-1-yl)-methyl]-2-(1H-tetrazol-5-yl)-biphenyle beschrieben, welche im Benzimidazolring durch eine Alkanoylaminomethylgruppe substituiert sind und Angiotensin-II-Antagonisten darstellen.

Es wurde nun gefunden, daß die neuen Benzimidazole der allgemeinen Formel

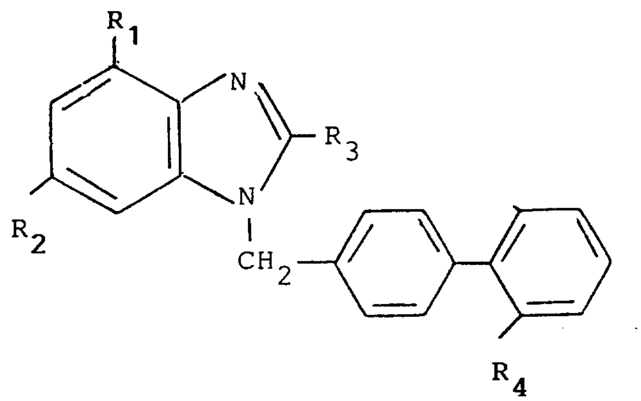

,(I)

deren Salze, insbesondere für die pharmazeutische Anwendung deren physiologisch verträglichen Salze mit anorganischen oder organischen Säuren oder Basen, noch wertvollere Angiotensin-Antagonisten, insbesondere Angiotensin-II-Antagonisten, darstellen.

In der obigen allgemeinen Formel I bedeutet
$R_1$ eine Methylgruppe oder ein Chloratom,
$R_2$ eine gegebenenfalls in 5- oder 6-Stellung durch ein Fluoratom substituierte 1-Methyl-benzimidazol-2-yl-gruppe, eine Pyridin-2-yl-, Chinolin-2-yl-, Isochinolin-1-yl-, Isochinolin-3-yl-, 8-Methyl-imidazo[1,2-a]pyridin-2-yl-, 5,6,7,8-Tetrahydro-imidazo[1,2-a]pyridin-2-yl- oder Imidazo[1,2-a]pyrimidin-2-yl-gruppe,
$R_3$ eine Ethyl-, n-Propyl-, n-Butyl-, Cyclopropyl- oder Cyclobutylgruppe und
$R_4$ Carboxy- oder Tetrazol-5-yl-gruppe.

Bevorzugte Verbindungen der obigen allgemeinen Formel I sind diejenigen, in denen
(i) $R_1$ eine Methylgruppe oder ein Chloratom,
$R_2$ eine 1-Methyl-benzimidazol-2-yl-gruppe,
$R_3$ eine n-Propyl-, Cyclopropyl- oder Cyclobutylgruppe und
$R_4$ eine Carboxy- oder Tetrazol-5-yl-gruppe oder
(ii) $R_1$ eine Methylgruppe,
$R_2$ eine 5,6,7,8-Tetrahydro-imidazo[1,2-a]pyridin-2-yl-gruppe,
$R_3$ eine Ethyl-, n-Propyl- oder n-Butylgruppe und
$R_4$ eine Carboxy- oder Tetrazol-5-y-gruppe oder
(iii) $R_1$ eine Methylgruppe,
$R_2$ eine 8-Methyl-imidazo[1,2-a]pyridin-2-yl-, Pyridin-2-yl-, Chinolin-2-yl-, Isochinolin-1-yl- oder Isochinolin-3-yl-gruppe,
$R_3$ eine n-Propylgruppe und
$R_4$ eine Carboxy- oder Tetrazol-5-yl-gruppe oder
(iv) $R_1$ eine Methylgruppe,
$R_2$ eine Imidazo[1,2-a]pyrimidln-2-yl-gruppe,
$R_3$ n-Propylgruppe und
$R_4$ eine Tetrazol-5-yl-gruppe oder
(v) $R_1$ eine Methylgruppe,
$R_2$ eine 1-Methyl-5-fluor-benzimidazol-2-yl- oder 1-Methyl-6-fluor-benzimidazol-2-yl-gruppe,
$R_3$ eine n-Propylgruppe und
$R_4$ eine Carboxygruppe bedeuten,
und deren Salze.

Besonders bevorzugte Verbindungen der obigen allgemeinen Formel I sind

(a) 4'-[(2-Cyclopropyl-4-methyl-6-(1-methylbenzimidazol-2-yl)-benzimidazol-1-yl)-methyl]-biphenyl-2-carbonsäure,

(b) 4'-[(2-n-Propyl-4-methyl-6-(1-methyl-5-fluor-benzimidazol-2-yl)-benzimidazol-1-yl)-methyl]-biphenyl-2-carbonsäure,

(c) 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,

(d) 4'-[(2-n-Propyl-4-methyl-6-(5,6,7,8-tetrahydro-imidazo[1,2-a]pyridin-2-yl)-benzimidazol-1-yl)-methyl]-biphenyl-2-carbonsäure,

(e) 4'-[(2-Ethyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl,

(f) 4'-[2-Ethyl-4-methyl-6-(5,6,7,8-tetrahydro-imidazo[1,2-a]pyridin-2-yl)-benzimidazol-1-yl)-methyl]-biphenyl-2-carbonsäure und

(g) 4'-[(2-n-Propyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl sowie

deren Salze, insbesondere deren physiologisch verträglichen Salze.

Erfindungsgemäß erhält man die Verbindungen nach folgenden Verfahren:

a) Cyclisierung einer Verbindung der allgemeinen Formel

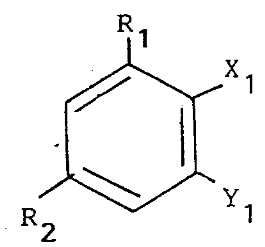

, (II)

in der

$R_1$ und $R_2$ wie eingangs definiert sind,

$Y_1$ eine Gruppe der allgemeinen Formel

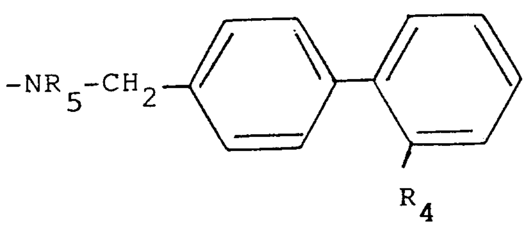

und $X_1$ eine $NH_2$-Gruppe oder eine Gruppe der allgemeinen Formel

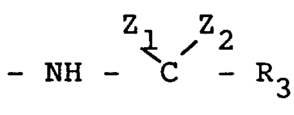

darstellen, wobei

$R_3$ und $R_4$ wie eingangs definiert sind,

$R_5$ ein Wasserstoffatom oder eine $R_3CO$-Gruppe, wobei $R_3$ wie vorstehend erwähnt definiert ist,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom,

eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250 ° C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitroaminoverbindung gegebenenfalls in Gegenwart einer Carbonsäure der allgemeinen Formel $R_3COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50 ° C, vorzugsweise jedoch bei Raumtemperatur durchgeführt.

b) Umsetzung eines Benzimidazols der allgemeinen Formel in der

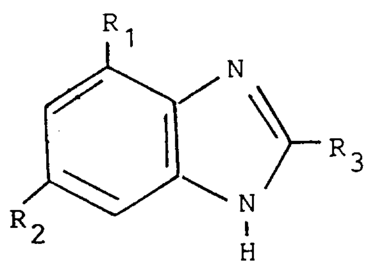

, (III)

$R_1$ bis $R_3$ wie eingangs definiert sind, mit einer Biphenylverbindung der allgemeinen Formel

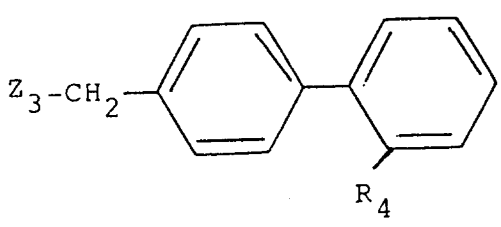

, (IV)

in der

$R_4$ wie eingangs definiert ist und

$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig

auch als Lösungsmittel verwendet werden können, vorzugsweise bei Temperaturen zwischen 0 und 100°C, z.B. bei Temperaturen zwischen Raumtemperatur und 50°C, durchgeführt.

Bei der Umsetzung erhält man vorzugsweise ein Gemisch der 1- und 3-Isomeren, welches gewünschtenfalls anschließend, vorzugsweise chromatographisch unter Verwendung eines Trägers wie Kieselgel oder Aluminiumoxid, oder durch Kristallisation in das entsprechende 1- und 3-Isomere aufgetrennt wird.

c) Zur Herstellung einer Verbindung der allgemeinen Formel I, in der $R_4$ eine Carboxygruppe darstellt: Überführung einer Verbindung der allgemeinen Formel

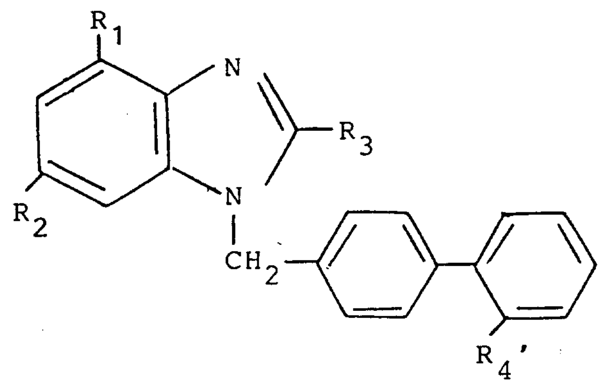

, (V)

in der

$R_1$ bis $R_3$ wie eingangs definiert sind und

$R_4$' eine mittels Hydrolyse, Thermolyse oder Hydrogenolyse in eine Carboxygruppe überführbare Gruppe darstellen.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4$' in einer Verbindung der allgemeinen Formel V eine Cyano- oder Aminocarbonylgruppe, so können diese Gruppen auch mit einem Nitrit, z.B. Natriumnitrit, in Gegenwart einer Saure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Bedeutet $R_4$' in einer Verbindung der allgemeinen Formel V beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_4$' in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Was-

serstoffatome, z.B. ein Halogenatom durch ein Wasserstoffatom, ersetzt werden.

d) Zur Herstellung einer Verbindung der allgemeinen Formel I, in der $R_4$ eine 1H–Tetrazolylgruppe darstellt:

Abspaltung eines Schutzrestes von einer Verbindung der allgemeinen Formel

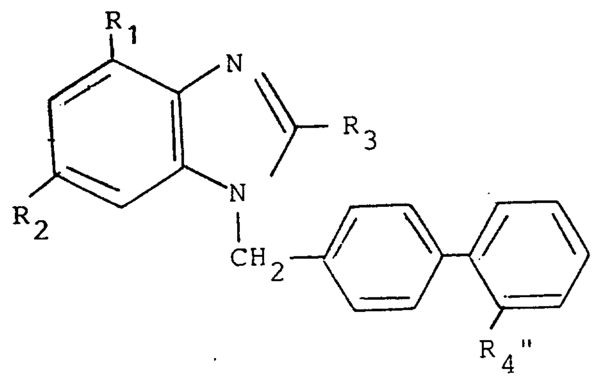

,(VI)

in der

$R_1$, $R_2$ und $R_3$ wie eingangs definiert sind und

$R_4''$ eine in 1– oder 3–Stellung durch einen Schutzrest geschützte 1H–Tetrazolylgruppe darstellt.

Als Schutzrest kommt beispielsweise die Triphenylmethyl–, Tributylzinn– oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_4$ eine 1H–Tetrazolylgruppe darstellt:

Umsetzung einer Verbindung der allgemeinen Formel

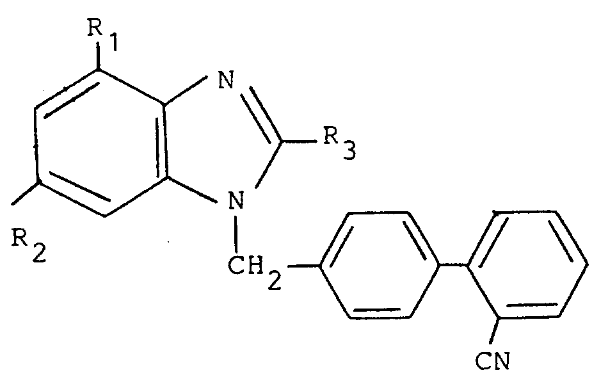

,(VII)

in der

$R_1$ bis $R_3$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid

hergestellt werden, erhaltene Tetrazolidsalz anschließend durch Ansäuern mit einer verdünnten Säure wie 2N Salzsäure oder 2N Schwefelsäure freigesetzt.

f) Zur Herstellung von Verbindungen der allgemeinen Formel I, in der $R_2$ eine Imidazo[1,2-a]pyridin-2-yl- oder Imidazo[1,2-a]pyrimidin-2-yl-gruppen darstellt:
Umsetzung einer Verbindung der allgemeinen Formel

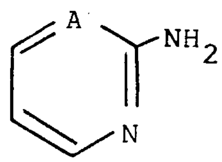

,(VIII)

in der
A eine durch eine Methylgruppe substituierte Methingruppe oder ein Stickstoffatom darstellt, mit einer Verbindung der allgemeinen Formel

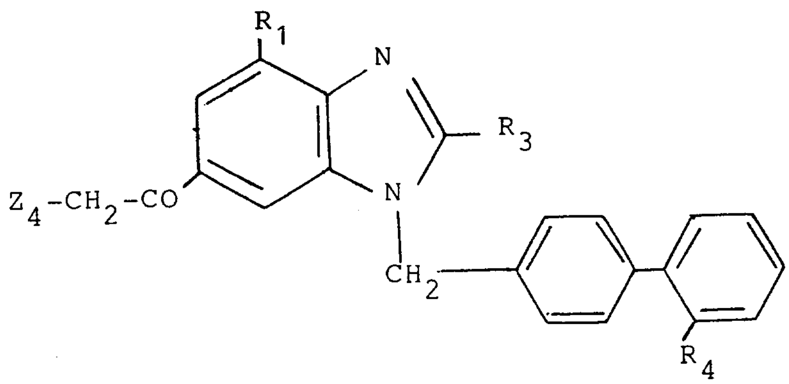

,(IX)

in der
$R_1$, $R_3$ und $R_4$ wie eingangs definiert sind und
$Z_4$ eine nukleophile Austrittsgruppe wie ein Halogenatom,
z.B. ein Chlor- oder Bromatom, oder eine Alkylsulfonyloxy- oder Arylsulfonyloxygruppe darstellt.

Die Umsetzung wird zweckmäßigerweise in einem Lösungsmittel oder Lösungsmittelgemisch wie Ethanol, Isopropanol, Benzol, Glykol, Glykolmonomethylether, Dimethylformamid, Dioxan oder Aceton beispielsweise bei Temperaturen zwischen 0 und 150°C, vorzugsweise bei Temperaturen zwischen 20 und 100°C, durchgeführt. Die Umsetzung kann jedoch auch ohne Lösungsmittel durchgeführt werden.

g) Zur Herstellung von Verbindungen der allgemeinen Formel I, in der $R_2$ eine der eingangs erwähnten Benzimidazol-2-yl-gruppen darstellt:
Cyclisierung einer Verbindung der allgemeinen Formel

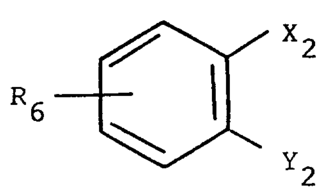

,(X)

in der
$R_6$ ein Wasserstoffatom oder in 5- oder 6-Stellung ein Fluoratom,
einer der Reste $X_2$ oder $Y_2$ eine $R_7$-NH-Gruppe und
der andere der Reste $X_2$ oder $Y_2$ eine Gruppe der allgemeinen Formel

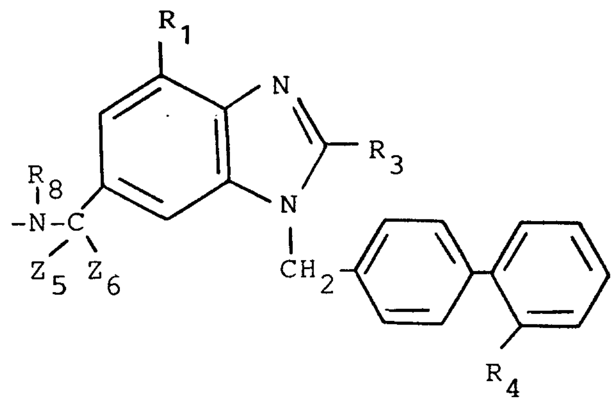

darstellen, wobei

wobei $R_1$, $R_3$ und $R_4$ wie eingangs definiert sind, einer der Reste $R_7$ oder $R_8$ ein Wasserstoffatom und der andere der Reste $R_7$ oder $R_8$ eine Methylgruppe,

$Z_5$ und $Z_6$, die gleich oder verschieden sein können, gegebenenfalls substituierte Aminogruppen oder gegebenenfalls durch niedere Alkylgruppen substituierte Hydroxy- oder Mercaptogruppen oder

$Z_5$ und $Z_6$, zusammen ein Sauerstoff- oder Schwefelatom,

eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, und gegebenenfalls anschließende Hydrolyse.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel X verwendeten Acylierungsmittel, z.B. in dem entsprechenden Nitril, Anhydrid, Säurehalogenid, Ester oder Amid, beispielsweise bei Temperaturen zwischen 0 und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel X im Reaktionsgemisch durch Reduktion einer entsprechenden o-Nitroaminoverbindung gegebenenfalls in Gegenwart einer Carbonsäure der allgemeinen Formel

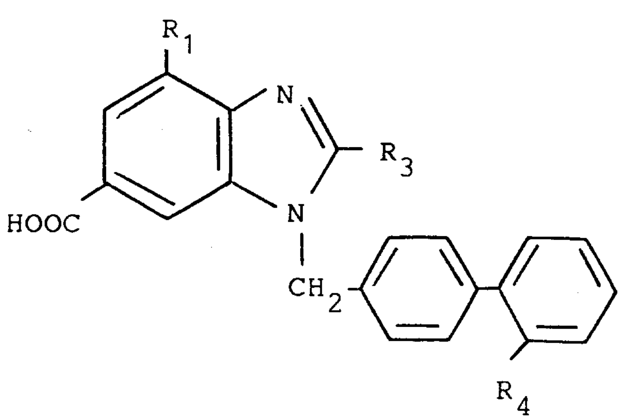

,(XI)

in der

$R_1$, $R_3$ und $R_4$ wie eingangs definiert sind, oder durch Acylierung einer entsprechenden o-Diaminoverbindung mit einer Carbonsäure der allgemeinen Formel XI hergestellt wird.

Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid

wird anschließend mittels Reduktion in eine entsprechende Verbindung der allgemeinen Formel I übergeführt.

Die anschließende Reduktion eines so erhaltenen N-Oxids wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50 °C, vorzugsweise jedoch bei Raumtemperatur durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bei den vorstehend beschriebenen Umsetzungen können gegebenenfalls vorhandene reaktive Gruppen wie Hydroxygruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches.

Ein gegebenenfalls so erhaltenes Isomerengemisch einer Verbindung der allgemeinen Formel I kann gewünschtenfalls vorzugsweise chromatographisch unter Verwendung eines Trägers wie Kieselgel oder Aluminiumoxid oder durch Kristallisation getrennt werden.

Desweiteren können die erhaltenen Verbindungen der allgemelnen Formel I können in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XI sind teilweise literaturbekannt oder man erhält diese nach literaturbekannten Verfahren.

So erhält man beispielsweise eine Verbindung der allgemeinen Formel II durch Alkylierung einer entsprechenden o-Aminonitroverbindung und anschließende Reduktion der Nitrogruppe.

Eine als Ausgangsstoff verwendete Verbindung der allgemeinen Formeln III, V, VI, VII, IX oder X erhält man durch Acylierung eines entsprechenden o-Phenylendiamins oder einer entsprechenden o-Aminonitroverbindung, anschließender Reduktion der Nitrogruppe und anschließender Cyclisierung einer so erhaltenen o-Diaminophenylverbindung und gegebenenfalls anschließender Abspaltung eines verwendeten Schutzrestes oder durch Cyclisierung eines entsprechend substituierten Benzimidazols mit einem entsprechenden Amin oder durch NH-Alkylierung eines entsprechenden 1H-Benzimidazols, wobei das so erhaltene Isomerengemisch anschließend mittels üblicher Methoden, z.B. mittels Chromatographie, aufgetrennt werden kann. Die vorstehend erwähnten Ausgangsverbindungen werden teilweise in der EP-A-0 392 317 beschrieben bzw. können den dort beschriebenen Verfahren hergestellt werden.

Beispielsweise erhält man 2-n-Propyl-4-methyl-6-(imidazo[1,2-a]pyridin-2-yl)-1H-benzimidazol durch Umsetzung von 4-Amino-3-methyl-acetophenon mit Buttersäurechlorid, anschließender Nitrierung, Bromierung, Ringschluß mit 2-Aminopyridin zu dem 6-n-Butanoylamido-3-(imidazo[1,2-a]pyridin-2-yl)-5-methyl-nitrobenzol, welches anschließend nach Reduktion der Nitrogruppe mittels Cyclisierung in die gewünschte Verbindung übergeführt wird oder

2-Cyclopropyl-4-methyl-6-(1-methylbenzimidazol-2-yl)-1H-benzimidazol durch Nitrierung von 3-Methyl-4-cyclopropylcarbonylamido-benzoesäuremethylester, anschließender Reduktion der Nitrogruppe und Cyclisierung zu 2-Cyclopropyl-4-methyl-6-methoxycarbonyl-1H-benzimidazol, welches anschließend mit 2-Methylamino-anilin unter Cyclisierung in die gewünschte Verbindung übergeführt wird.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[(2-Cyclopropyl-4-methyl-6-(1-methylbenzimidazol-2-yl)-benzimidazol-1-yl)-methyl]-biphenyl-2-carbonsäure,

B = 4'-[(2-n-Propyl-4-methyl-6-(1-methyl-5-fluor-benzimidaZol-2-yl)-benzimidazol-1-yl)-methyl]-biphenyl-2-carbonsäure,

C = 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,

D = 4'-[(2-n-Propyl-4-methyl-6-(5,6,7,8-tetrahydro-imidazo[1,2-a]pyridin-2-yl)-benzimidazol-1-yl)-methyl]-biphenyl-2-carbonsäure,

E = 4'-[(2-n-Propyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl und

F = 4'[(2-n-Propyl-4-chlor-6-(1-methylbenzimidazol-2-yl)-benzimidazol-1-yl)-methyl]-2-(1H-tetrahol-5-yl)-biphenyl-hydrochlorid,

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$125^I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis F zeigen in dem beschriebenen Test folgende $IC_{50}$-Werte:

| Substanz | $IC_{50}$ [nM] |
|---|---|
| A | 12,0 |
| B | 26,0 |
| C | 3,4 |
| D | 1,2 |
| E | 1,7 |
| F | 20,0 |

Zusätzlich wurden die Verbindungen A und C an wachen, renal hypertensiven Ratten auf ihre Wirkung nach oraler Gabe nach literaturbekannten Methoden getestet. Bei einer Dosis von 10 mg/kg zeigten diese Verbindungen eine blutdrucksenkende Wirkung.

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Präven-

tion von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

## Beispiel 1

### 4'-[(2-Cyclopropyl-4-methyl-6-(1-methylbenzimidazol-2-yl)-benzimidazol-1-yl)-methyl]-biphenyl-2-carbonsäure

a) 4'-[(2-Cyclopropyl-4-methyl-6-(1-methylbenzimidazol-2-yl)-benzimidazol-1-yl)-methyl]-biphenyl-2-carbonsäure-tert.butylester

Zu einer Lösung von 5,1 g (17 mMol) 2-Cyclopropyl-4-methyl-6-(1-methylbenzimidazol-2-yl)-benzimidazol in 60 ml absolutem Dimethylformamid werden bei Raumtemperatur 2,24 g (20 mMol) Kalium-tert.butylat gegeben und 30 Minuten lang gerührt. Dann wird eine Lösung von 7,0 g (20 mMol) 4'-Brommethyl-biphenyl-2-carbonsäure-tert.butylester in 25 ml absolutem Dimethylformamid zugetropft und nach beendeter Zugabe weitere 90 Minuten lang bei Raumtemperatur gerührt. Anschließend wird das Gemisch in ca. 200 ml gesättigter Natriumchlorid-Lösung gegossen, das danach ausgefallene Rohprodukt abgesaugt, mit Wasser gewaschen, an der Luft getrocknet und durch Säulenchromatographie (500 g Kieselgel, Laufmittel: Dichlormethan mit 2,5 % Ethanol) gereinigt.
Ausbeute: 8,2 g (85 % der Theorie),
$R_f$-Wert: 0,64 (Kieselgel; Dichlormethan/Ethanol = 19:1).

b) 4'-[(2-Cyclopropyl-4-methyl-6-(1-methylbenzimidazol-2-yl)-benzimidazol-1-yl)-methyl]-biphenyl-2-carbonsäure

Zu einer Lösung von 8,2 g (14,4 mMol) 4'-[(2-Cyclopropyl-4-methyl-6-(1-methylbenzimidazol-2-yl)-benzimidazol-1-yl)-methyl]-biphenyl-2-carbonsäure-tert.butylester in 90 ml Dichlormethan werden 45 ml Trifluoressigsäure gegeben und 2 Stunden bei Raumtemperatur gerührt. Danch wird das Gemisch zur Trockne eingedampft, der Rückstand in 200 ml Essigester gelöst und unter heftigem Rühren durch Zugabe von Natronlauge neutralisiert. Das dabei auskristallisierte Rohprodukt wird abgesaugt und durch Säulenchromatographie (400 g Kieselgel; Laufmittel: Dichlormethan mit 3 bis 5 % Ethanol) gereinigt.
Ausbeute: 3,9 g (52 % der Theorie),
Schmelzpunkt: 244-246 °C

$C_{33}H_{28}N_4O_2$ (512,60)

| | | | |
|---|---|---|---|
| Ber.: | C 77,32 | H 5,51 | N 10,93 |
| Gef.: | 77,75 | 5,71 | 10,94 |

Beispiel 2

4' – [(2 – Cyclopropyl – 4 – methyl – 6 – (1 – methylbenzimidazol – 2 – yl) – benzimidazol – 1 – yl) – methyl] – 2 – (1H – tetrazol – 5 – yl) – biphenyl

a) 4' – [(2 – Cyclopropyl – 4 – methyl – 6 – (1 – methylbenzimidazol – 2 – yl) – benzimidazol – 1 – yl) – methyl] – 2 – cyano – biphenyl

Zu einer Lösung von 6,6 g (22 mMol) 2 – Cyclopropyl – 4 – methyl – 6 – (1 – methylbenzimidazol – 2 – yl) – benzimidazol in 60 ml absolutem Dimethylformamid werden bei Raumtemperatur 2,9 g (26 mMol) Kalium – tert.butylat gegeben und 30 Minuten lang gerührt. Dann wird eine Lösung von 7,1 g (26 mMol) 4' – Brommethyl – biphenyl – 2 – carbonsäurenitril in 25 ml absolutem Dimethylformamid zugetropft und weitere 2 Stunden bei Raumtemperatur gerührt. Anschließend wird das Gemisch in ca. 200 ml gesättigte Natriumchlorid – Lösung gegossen, das danach ausgefallene Rohprodukt abgesaugt, mit Wasser gewa – schen, an der Luft getrocknet und durch Säulenchromatographie (800 g Kieselgel; Laufmittel; Dichlormethan mit 3 bis 4 % Ethanol) gereinigt.
Ausbeute: 5,5 g (51 % der Theorie),
$R_f$ – Wert: 0,60 (Kieselgel; Dichlormethan/Ethanol = 19:1).

b) 4' – [(2 – Cyclopropyl – 4 – methyl – 6 – (1 – methylbenzimidazol – 2 – yl) – benzimidazol – 1 – yl) – methyl] – 2 – (1H – tetrazol – 5 – yl) – biphenyl

Ein Gemisch aus 5,5 g (11 mMol) 4' – [(2 – Cyclopropyl – 4 – methyl – 6 – (1 – methylbenzimadazol – 2 – yl) – benzimidol – 1 – yl) – methyl] – 2 – cyano – biphenyl, 14,6 g (275 mMol) Ammoniumchlorid, 17,9 g (275 mMol) Natriumazid und 200 ml Dimethylformamid wird 20 Stunden lang bei 140 ° C gerührt, dann nach dem Abkühlen in ca. 400 ml kalt gesättigte Natriumchlorid – Lösung eingerührt. Das dabei ausgefallene Rohr – produkt wird abgesaugt, mit Wasser gewaschen, getrocknet und schließlich durch Säulenchromatographie (400 g Kieselgel; Laufmittel: Dichlormethan mit 2 bis 5 % Ethanol) gereinigt.
Ausbeute: 3,5 g (59 % der Theorie),
Schmelzpunkt: 245 – 247 ° C
$C_{33}H_{28}N_8$ (536,65)

| | | | |
|---|---|---|---|
| Ber.: | C 73,86 | H 5,26 | N 20,88 |
| Gef.: | 73,95 | 5,42 | 20,90 |

Beispiel 3

4' – [(2 – Cyclobutyl – 4 – methyl – 6 – (1 – methylbenzimidazol – 2 – yl) – benzimidazol – 1 – yl) – methyl] – biphenyl – 2 – carbonsäure

Hergestellt analog Beispiel 1 aus 4' – [(2 – Cyclobutyl – 4 – methyl – 6 – (1 – methylbenzimidazol – 2 – yl) – benzimidazol – 1 – yl) – methyl] – biphenyl – 2 – carbonsäure – tert.butylester und Trifluoressigsäure in Methylenchlorid.
Ausbeute: 63 % der Theorie,
Schmelzpunkt: 189 – 191 ° C
$C_{34}H_{30}N_4O_2$ (526,60)

| | | | |
|---|---|---|---|
| Ber.:<br>Gef.: | C 77,55<br>77,35 | H 5,74<br>5,92 | N 10,64<br>10,40 |

Beispiel 4

4'-[(2-Cyclobutyl-4-methyl-6-(1-methylbenzimidazol-2-yl)-benzimidazol-1-yl)-methyl]-2-(1H-tetrazol-5-yl)-biphenyl

Hergestellt analog Beispiel 2 aus 4'-[(2-Cyclobutyl-4-methyl-6-(1-methylbenzimidazol-2-yl)-benzimidazol-1-yl)-methyl]-2-cyano-biphenyl und Natriumazid in Dimethylformamid.
Ausbeute: 61 % der Theorie,
Schmelzpunkt: 197-199 °C
$C_{34}H_{30}N_8$ (550,70)

| | | | |
|---|---|---|---|
| Ber.:<br>Gef.: | C 74,16<br>74,12 | H 5,49<br>5,74 | N 20,35<br>20,67 |

Beispiel 5

4'-[(2-n-Propyl-4-methyl-6-(1-methyl-5-fluor-benzimidazol-2-yl)-benzimidazol-1-yl)-methyl]-biphenyl-2-carbonsäure

Hergestellt analog Beispiel 1 aus 4'-[(2-n-Propyl-4-methyl-6-(1-methyl-5-fluor-benzimidazol-2-yl)-benzimidazol-1-yl)-methyl]-biphenyl-2-carbonsäure-tert.butylester und Trifluoressigsäure in Methylenchlorid.
Ausbeute: 34 % der Theorie,
Schmelzpunkt: 250-252 °C
$C_{33}H_{29}FN_4O_2$ (532,60)

| | | | |
|---|---|---|---|
| Ber.:<br>Gef.: | C 74,42<br>74,14 | H 5,49<br>5,64 | N 10,52<br>10,54 |

Analog Beispiel 5 werden folgende Verbindungen erhalten:
4'-[(2-n-Propyl-4-methyl-6-(pyridin-2-yl)-benzimidazol-1-yl)-methyl]-biphenyl-2-carbonsäure
4'-[(2-n-Propyl-4-methyl-6-(chinolin-2-yl)-benzimidazol-1-yl)-methyl]-biphenyl-2-carbonsäure
4'-[(2-n-Propyl-4-methyl-6-(isochinolin-3-yl)-benzimidazol-1-yl)-methyl]-biphenyl-2-carbonsäure
4'-[(2-n-Propyl-4-methyl-6-(isochinolin-1-yl)-benzimidazol-1-yl)-methyl]-biphenyl-2-carbonsäure

Beispiel 6

4'-[(2-n-Propyl-4-methyl-6-(imidazo[1,2-a]pyrimidin-2-yl)-benzimidazol-1-yl)-methyl]-2-(1H-tetrazol-5-yl)-biphenyl

Hergestellt analog Beispiel 2 aus 4'-[(2-n-Propyl-4-methyl-6-(imidazo[1,2-a]pyrimidin-2-yl)-benzimidazol-1-yl)-methyl]-2-cyano-biphenyl und Natriumazid in Dimethylformamid.
Ausbeute: 16,5 % der Theorie,
Schmelzpunkt: ab 275 °C (Zers.)
$C_{31}H_{27}N_9 \times H_2O$ (543,65)

| | | | |
|---|---|---|---|
| Ber.: | C 68,49 | H 5,38 | N 23,19 |
| Gef.: | 68,25 | 5,50 | 23,37 |

Analog Beispiel 6 werden folgende Verbindungen erhalten:
4' – [(2 – n – Propyl – 4 – methyl – 6 – (chinolin – 2 – yl) – benzimidazol – 1 – yl) – methyl] – 2 – (1H – tetrazol – 5 – yl) – biphenyl
4' – [(2 – n – Propyl – 4 – methyl – 6 – (isochinolin – 3 – yl) – benzimidazol – 1 – yl) – methyl] – 2 – (1H – tetrazol – 5 – yl) – biphenyl
4' – [(2 – n – Propyl – 4 – methyl – 6 – (isochinolin – 1 – yl) – benzimidazol – 1 – yl) – methyl] – 2 – (1H – tetrazol – 5 – yl) – biphenyl

## Beispiel 7

**4' – [(2 – n – Propyl – 4 – methyl – 6 – (5,6,7,8 – tetrahydro – imidazo[1,2 – a]pyridin – 2 – yl) – benzimidazol – 1 – yl) – methyl] – biphenyl – 2 – carbonsäure**

Hergestellt analog Beispiel 1 aus 4' – [(2 – n – Propyl – 4 – methyl – 6 – (5,6,7,8 – tetrahydro – imidazo – [1,2 – a] – pyridin – 2 – yl) – benzimidazol – 1 – yl) – methyl] – biphenyl – 2 – carbonsäure – tert.butylester und Trifluoressigsäure in Methylenchlorid.
Ausbeute: 67 % der Theorie,
Schmelzpunkt: ab 240 ° C (sintern)
$C_{32}H_{32}N_4O_2$ (504,64)

| | | | |
|---|---|---|---|
| Ber.: | C 76,16 | H 6,39 | N 11,10 |
| Gef.: | 75,94 | 6,46 | 11,20 |

Analog Beispiel 7 wird folgende Verbindung erhalten:
4' – [(2 – n – Butyl – 4 – methyl – 6 – (5,6,7,8 – tetrahydro – imidazo[1,2 – a] – pyridin – 2 – yl) – benzimidazol – 1 – yl) – methyl] – biphenyl – 2 – carbonsäure

## Beispiel 8

**4' – [(2 – n – Propyl – 4 – methyl – 6 – (5,6,7,8 – tetrahydro – imidazo[1,2 – a] – pyridin – 2 – yl) – benzimidazol – 1 – yl) – methyl] – 2 – (1H – tetrazol – 5 – yl) – biphenyl**

Hergestellt analog Beispiel 2 aus 4' – [(2 – n – Propyl – 4 – methyl – 6 – (5,6,7,8 – tetrahydro – imidazo – [1,2 – a] – pyridin – 2 – yl) – benzimidazol – 1 – yl) – methyl] – 2 – cyano – biphenyl und Natriumazid in Dimethylformamid.
Ausbeute: 73,5 % der Theorie,
Schmelzpunkt: ab 275 ° C (Zers.)
$C_{32}H_{32}N_8$ (528,67)

| | | | |
|---|---|---|---|
| Ber.: | C 72,70 | H 6,10 | N 21,20 |
| Gef.: | 72,40 | 6,07 | 21,48 |

Analog Beispiel 8 wird folgende Verbindung erhalten:
4' – [(2 – n – Butyl – 4 – methyl – 6 – (5,6,7,8 – tetrahydro – imidazo[1,2 – a] – pyridin – 2 – yl) – benzimidazol – 1 – yl) – methyl] – 2 – (1H – tetrazol – 5 – yl) – biphenyl

Beispiel 9

4'-[(2-n-Propyl-4-methyl-6-(1-methyl-6-fluor-benzimidazol-2-yl)-benzimidazol-1-yl)-methyl]-biphenyl-2-carbonsäure

Hergestellt analog Beispiel 1 aus 4'-[(2-n-Propyl-4-methyl-6-(1-methyl-6-fluor-benzimidazol-2-yl)-benzimidazol-1-yl)-methyl]-biphenyl-2-carbonsäure-tert.butylester und Trifluoressigsäure in Methylenchlorid.
Ausbeute: 76 % der Theorie,
Schmelzpunkt: 243-245 °C
$C_{33}H_{29}FN_4O_2$ (532,60)

| | | | |
|---|---|---|---|
| Ber.: | C 74,42 | H 5,49 | N 10,52 |
| Gef.: | 74,74 | 5,52 | 10,77 |

Massenspektrum: m/e = 532

Beispiel 10

4'-[(2-n-Propyl-4-chlor-6-(1-methylbenzimidazol-2-yl)-benzimidazol-1-yl)-methyl]-biphenyl-2-carbonsäure

Hergestellt analog Beispiel 1 aus 4'-[(2-n-Propyl-4-chlor-6-(1-methylbenzimidazol-2-yl)-benzimidazol-1-yl)-methyl]-biphenyl-2-carbonsäure-tert.butylester und Trifluoressigsäure in Methylenchlorid.
Ausbeute: 52,7 % der Theorie,
Schmelzpunkt: 292-295 °C
$C_{32}H_{27}ClN_4O_2$ (535,06)
$R_f$-Wert: 0,30 (Kieselgel; Methylenchlorid/Ethanol = 19:1)

| | | | | |
|---|---|---|---|---|
| Ber.: | C 71,90 | H 5,08 | N 10,45 | Cl 6,63 |
| Gef.: | 71,29 | 5,21 | 10,40 | 6,76 |

Beispiel 11

4'-[(2-n-Propyl-4-chlor-6-(1-methylbenzimidazol-2-yl)-benzimidazol-1-yl)-methyl]-2-(1H-tetrazol-5-yl)-biphenyl-hydrochlorid

Hergestellt analog Beispiel 2 aus 4'-[(2-n-Propyl-4-chlor-6-(1-methylbenzimidazol-2-yl)-benzimidazol-1-yl)-methyl]-2-cyano-biphenyl und Natriumazid in Dimethylformamid.
Ausbeute: 54,8 % der Theorie,
Schmelzpunkt: ab 204 °C sintern
$C_{32}H_{27}ClN_8$ x HCl (595,55)
$R_f$-Wert: 0,20 (Kieselgel; Petrolether/Essigester = 1:1 und 1 % Eisessig)

| | | | | |
|---|---|---|---|---|
| Ber.: | C 62,55 | H 4,71 | N 18,85 | Cl 11,85 |
| Gef.: | 62,34 | 4,97 | 18,84 | 11,57 |

Beispiel 12

4'-[(2-Ethyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl

Hergestellt analog Beispiel 2 aus 4'-[(2-Ethyl-4-methyl-6-(5,6,7,8-tetrahydro-imidazo[1,2-a]-pyridin-2-yl)-benzimidazol-1-yl)-methyl]-2-cyano-biphenyl und Natriumazid in Dimethylformamid.
Ausbeute: 21 % der Theorie,
Schmelzpunkt: amorph
$R_f$-Wert: 0,27 (Kieselgel; Methylenchlorid/Ethanol = 9:1)
$C_{31}H_{30}N_8$ (514,64)

| | | | |
|---|---|---|---|
| Ber.: | C 72,35 | H 5,88 | N 21,78 |
| Gef.: | 72,01 | 5,82 | 21,44 |

Beispiel 13

4'-[(2-n-Propyl-4-methyl-6-(8-methyl-imidazo[1,2-a]pyridin-2-yl)-benzimidazol-1-yl)-methyl]-biphenyl-2-carbonsäure

Hergestellt analog Beispiel 1 aus 4'-[(2-n-Propyl-4-methyl-6-(8-methyl-imidazo[1,2-a]-pyridin-2-yl)-benzimidazol-1-yl)-methyl]-biphenyl-2-carbonsäure-tert.butylester und Trifluoressigsäure in Methylenchlorid.
Ausbeute: 87 % der Theorie,
Schmelzpunkt: 295-297°C
$R_f$-Wert: 0,34 (Kieselgel; Methylenchlorid/Ethanol = 9:1)
$C_{33}H_{30}N_4O_2 \times H_2O$ (532,65)

| | | | |
|---|---|---|---|
| Ber.: C | 74,41 | H 6,06 | N 10,52 |
| Gef.: | 74,81 | 6,05 | 10,43 |

Beispiel 14

4'-[(2-n-Propyl-4-methyl-6-(2-pyridyl)-benzimidazol-1-yl)-methyl]-2-(1H-tetrazol-5-yl)-biphenyl

Hergestellt analog Beispiel 2 aus 4'-[(2-n-Propyl-4-methyl-6-(2-pyridyl)-benzimidazol-1-yl)-methyl]-2-cyano-biphenyl und Natriumazid in Dimethylformamid.
Ausbeute: 56 % der Theorie,
Schmelzpunkt: ab 136°C (Zers.)
$C_{30}H_{27}N_7 \times 0,5\ H_2O$ (494,60)

| | | | |
|---|---|---|---|
| Ber.: | C 72,85 | H 5,71 | N 19,83 |
| Gef.: | 72,45 | 6,01 | 19,83 |

Beispiel 15

4' – [(2 – n – Propyl – 4 – methyl – 6 – (8 – methyl – imidazo[1,2 – a]pyridin – 2 – yl) – benzimidazol – 1 – yl) – methyl] – 2 – (1H – tetrazol – 5 – yl) – biphenyl

Hergestellt analog Beispiel 2 aus 4' – [(2 – n – Propyl – 4 – methyl – 6 – (8 – methyl – imidazo[1,2 – a] – pyridin – 2 – yl) – benzimidazol – 1 – yl) – methyl] – 2 – cyano – biphenyl und Natriumazid in Dimethylformamid.
Ausbeute: 19 % der Theorie,
Schmelzpunkt: amorph
$R_f$ – Wert 0,36 (Kieselgel; Methylenchlorid/Ethanol = 9:1)
$C_{33}H_{30}N_8$ (538,61)
Massenspektrum: m/e = 538

Beispiel 16

4' – [2 – Ethyl – 4 – methyl – 6 – (5,6,7,8 – tetrahydro – imidazo[1,2 – a]pyridin – 2 – yl) – benzimidazol – 1 – yl) – methyl] – biphenyl – 2 – carbonsäure

Hergestellt analog Beispiel 1 aus 4' – [2 – Ethyl – 4 – methyl – 6 – (5,6,7,8 – tetrahydro – imidazo[1,2 – a] – pyridin – 2 – yl) – benzimidazol – 1 – yl) – methyl] – biphenyl – 2 – carbonsäure – tert.butylester und Trifluoressigsäure in Methylenchlorid.
Ausbeute: 50 % der Theorie,
Schmelzpunkt: > 300 ° C
$R_f$ – Wert: 0,16 (Kieselgel; Methylenchlorid/Ethanol = 9:1)

Beispiel 17

Ampullen, enthaltend 50 mg Wirkstoff pro 5 ml

| | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel 18

Ampullen, enthaltend 100 mg Wirkstoff pro 5 ml

| | |
|---|---|
| Wirkstoff | 100 mg |
| Methylglucamin | 35 mg |
| Glykofurol | 1000 mg |
| Polyethylenglykol – Polypropylenglykol – Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel 19

Tabletten, enthaltend 50 mg Wirkstoff

| | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50°C getrocknet und erneut gesiebt.

Nach Zumischen des Schmiermittels wird das Granulat auf einer Tablettiermaschine verpresst.

Beispiel 20

Dragees, enthaltend 50 mg Wirkstoff

| | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel 21

Dragees, enthaltend 100 mg Wirkstoff

| | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel 22

Kapseln, enthaltend 250 mg Wirkstoff

| | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel 23

Orale Suspension, enthaltend 50 mg Wirkstoff pro 5 ml

| | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 ° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel 24

Suppositorien, enthaltend 100 mg Wirkstoff

| | |
|---|---|
| Wirkstoff | 100,0 mg |
| Adeps solidus | 1600,0 mg |
| | 1700,0 mg |

Herstellung:

Das Hartfett wird geschmolzen. Bei 40 ° C wird die gemahlene Wirksubstanz in der Schmelze homogen dispergiert. Es wird auf 38 ° C abgekühlt und in schwach vorgekühlte Suppositorienformen ausgegossen.

**Patentansprüche**

**1.** Benzimidazole der allgemeinen Formel

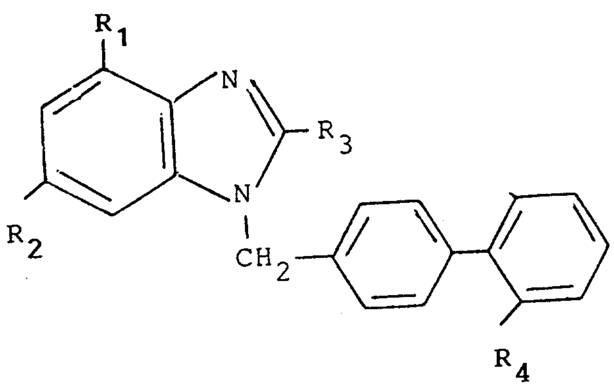

, (I)

in der
$R_1$ eine Methylgruppe oder ein Chloratom,
$R_2$ eine gegebenenfalls in 5- oder 6-Stellung durch ein Fluoratom substituierte 1-Methyl-benzimidazol-2-yl-gruppe, eine Pyridin-2-yl-, Chinolin-2-yl-, Isochinolin-1-yl-, Isochinolin-3-yl-, 8-Methyl-imidazo[1,2-a]pyridin-2-yl-, 5,6,7,8-Tetrahydro-imidazo[1,2-a]pyridin-2-yl- oder Imidazo[1,2-a]pyrimidin-2-yl-gruppe,
$R_3$ eine Ethyl-, n-Propyl-, n-Butyl-, Cyclopropyl- oder Cyclobutylgruppe und
$R_4$ Carboxy- oder Tetrazol-5-yl-gruppe bedeuten, und deren Salze.

**2.** Benzimidazole der allgemeinen Formel I gemäß Anspruch 1, in der
(i) $R_1$ eine Methylgruppe oder ein Chloratom,
$R_2$ eine 1-Methyl-benzimidazol-2-yl-gruppe,
$R_3$ eine n-Propyl-, Cyclopropyl- oder Cyclobutylgruppe und
$R_4$ eine Carboxy- oder Tetrazol-5-yl-gruppe oder
(ii) $R_1$ eine Methylgruppe,
$R_2$ eine 5,6,7,8-Tetrahydro-imidazo[1,2-a]pyridin-2-yl-gruppe,
$R_3$ eine Ethyl-, n-Propyl- oder n-Butylgruppe und

$R_4$ eine Carboxy- oder Tetrazol-5-y-gruppe oder
(iii) $R_1$ eine Methylgruppe,
$R_2$ eine 8-Methyl-imidazo[1,2-a]pyridin-2-yl-, Pyridin-2-yl-, Chinolin-2-yl-, Isochinolin-1-yl- oder Isochinolin-3-yl-gruppe,
$R_3$ eine n-Propylgruppe und
$R_4$ eine Carboxy- oder Tetrazol-5-yl-gruppe oder
(iv) $R_1$ eine Methylgruppe,
$R_2$ eine Imidazo[1,2-a]pyrimidin-2-yl-gruppe,
$R_3$ n-Propylgruppe und
$R_4$ eine Tetrazol-5-yl-gruppe oder
(v) $R_1$ eine Methylgruppe,
$R_2$ eine 1-Methyl-5-fluor-benzimidazol-2-yl- oder 1-Methyl-6-fluor-benzimidazol-2-yl-gruppe,
$R_3$ eine n-Propylgruppe und
$R_4$ eine Carboxygruppe bedeuten,
und deren Salze.

**3.** Folgende Benzimidazole der allgemeinen Formel I gemäß Anspruch 1:
(a) 4'-[(2-Cyclopropyl-4-methyl-6-(1-methylbenzimidazol-2-yl)-benzimidazol-1-yl)-methyl]-biphenyl-2-carbonsäure,
(b) 4'-[(2-n-Propyl-4-methyl-6-(1-methyl-5-fluor-benzimidazol-2-yl)-benzimidazol-1-yl)-methyl]-biphenyl-2-carbonsäure,
(c) 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,
(d) 4'-[(2-n-Propyl-4-methyl-6-(5,6,7,8-tetrahydro-imidazo[1,2-a]pyridin-2-yl)-benzimidazol-1-yl)-methyl]-biphenyl-2-carbonsäure,
(e) 4'-[(2-Ethyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl,
(f) 4'-[2-Ethyl-4-methyl-6-(5,6,7,8-tetrahydro-imidazo[1,2-a]pyridin-2-yl)-benzimidazol-1-yl)-methyl]-biphenyl-2-carbonsäure und
(g) 4'-[(2-n-Propyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl,
und deren Salze.

**4.** Folgende Benzimidazole der allgemeinen Formel I gemäß Anspruch 1:
(a) 4'-[(2-n-Propyl-4-methyl-6-(5,6,7,8-tetrahydro-imidazo[1,2-a]pyridin-2-yl)-benzimidazol-1-yl)-methyl]-biphenyl-2-carbonsäure,
(b) 4'-[(2-Ethyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl,
(c) 4'-[2-Ethyl-4-methyl-6-(5,6,7,8-tetrahydro-imidazo[1,2-a]pyridin-2-yl)-benzimidazol-1-yl)-methyl]-biphenyl-2-carbonsäure und
(d) 4'-[(2-n-Propyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl,
und deren Salze.

**5.** Physiologisch verträgliche Salze der Verbindungen nach mindestens einem der Ansprüche 1 bis 4 mit anorganischen oder organischen Säuren oder Basen.

**6.** Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

**7.** Verwendung einer Verbindung nach mindestens einem der Ansprüche 1 bis 5 zur Herstellung eines Arzneimittels mit Angiotensin-antagonistischer Wirkung.

**8.** Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

**9.** Verfahren zur Herstellung der Benzimidazole gemäß den Ansprüchen 1 bis 5, dadurch gekennzeichnet, daß

a) eine Verbindung der allgemeinen Formel

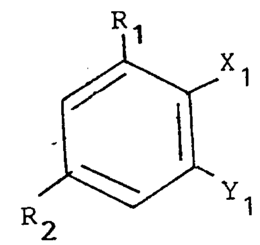

,(II)

in der
$R_1$ und $R_2$ wie in den Ansprüchen 1 bis 4 definiert sind,
$Y_1$ eine Gruppe der allgemeinen Formel

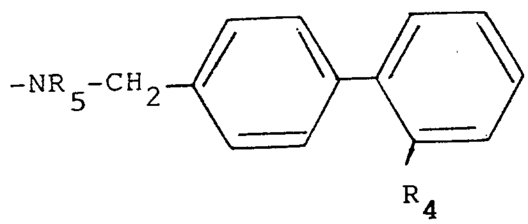

und $X_1$ eine $NH_2$-Gruppe oder eine Gruppe der allgemeinen Formel

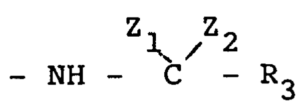

darstellen, wobei
$R_3$ und $R_4$ wie in den Ansprüchen 1 bis 4 definiert sind,
$R_5$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom,
eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten,
cyclisiert wird oder

b) ein Benzimidazol der allgemeinen Formel

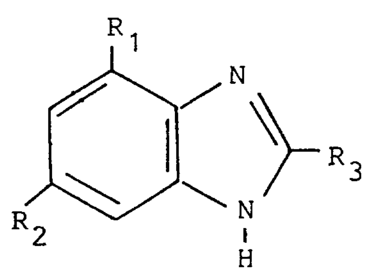

,(III)

in der
$R_1$ bis $R_3$ wie in den Ansprüchen 1 bis 4 definiert sind, mit einer Biphenylverbindung der

allgemeinen Formel

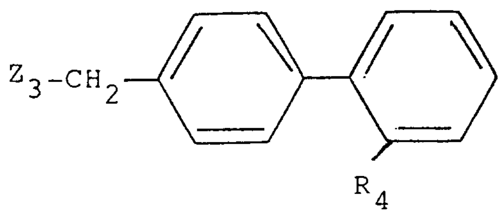

, (IV)

in der
$R_4$ wie in den Ansprüchen 1 bis 4 definiert ist und
$Z_3$ eine nukleophile Austrittsgruppe darstellt, umgesetzt wird oder
c) zur Herstellung einer Verbindung der allgemeinen Formel I, in der $R_4$ eine Carboxygruppe darstellt, eine Verbindung der allgemeinen Formel

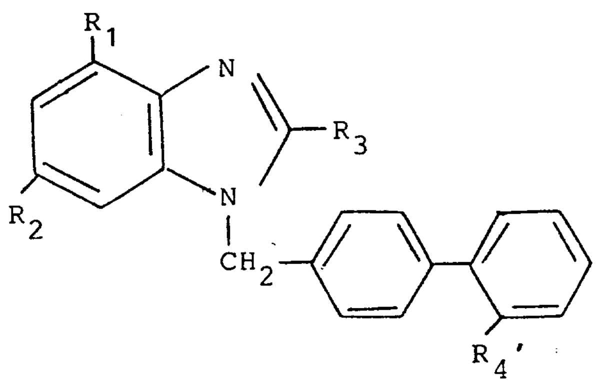

, (V)

in der
$R_1$ bis $R_3$ wie in den Ansprüchen 1 bis 4 definiert sind und
$R_4'$ eine mittels Hydrolyse, Thermolyse oder Hydrogenolyse in eine Carboxygruppe überführbare Gruppe darstellt, in eine entsprechende Carboxyverbindung übergeführt wird oder
d) zur Herstellung einer Verbindung der allgemeinen Formel I, in der $R_4$ eine 1H-Tetrazolylgruppe darstellt, ein Schutzrest von einer Verbindung der allgemeinen Formel

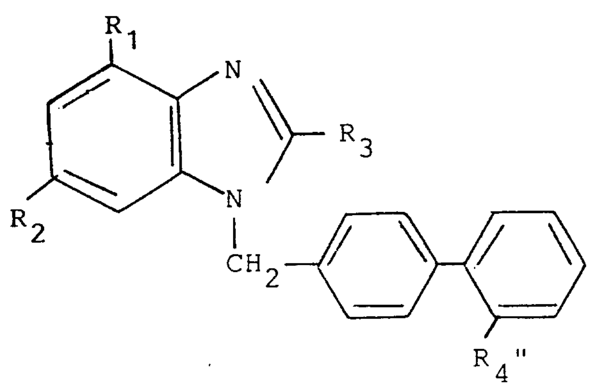

, (VI)

in der
$R_1$, $R_2$ und $R_3$ wie in den Ansprüchen 1 bis 4 definiert sind und
$R_4''$ eine in 1- oder 3-Stellung durch einen Schutzrest geschützte 1H-Tetrazolylgruppe darstellt, abgespalten wird oder
e) zur Herstellung einer Verbindung der allgemeinen Formel I, in der $R_4$ eine 1H-Tetrazolylgruppe darstellt, eine Verbindung der allgemeinen Formel

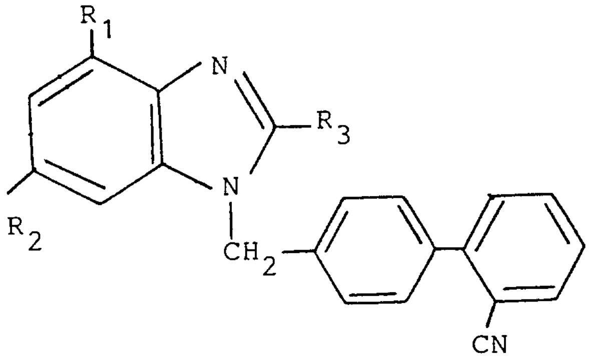

,(VII)

in der
$R_1$ bis $R_3$ wie in den Ansprüchen 1 bis 4 definiert sind, mit Stickstoffwasserstoffsäure oder deren Salzen umgesetzt wird oder
f) zur Herstellung von Verbindungen der allgemeinen Formel I, in der $R_2$ eine Imidazo[1,2-a]pyridin-2-yl- oder Imidazo[1,2-a]pyrimidin-2-yl-gruppen darstellt, eine Verbindung der allgemeinen Formel

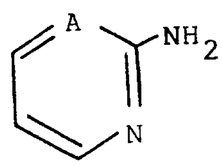

,(VIII)

in der
A eine durch eine Methylgruppe substituierte Methingruppe oder ein Stickstoffatom darstellt, mit einer Verbindung der allgemeinen Formel

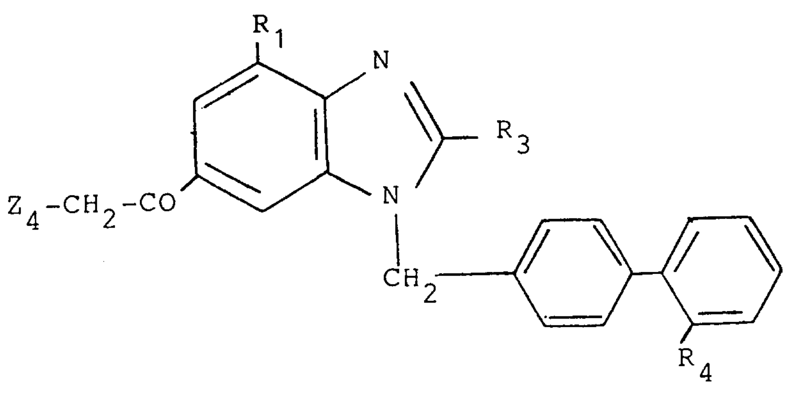

,(IX)

in der
$R_1$, $R_3$ und $R_4$ wie in den Ansprüchen 1 bis 4 definiert sind und
$Z_4$ eine nukleophile Austrittsgruppe darstellt, umgesetzt wird oder
g) zur Herstellung von Verbindungen der allgemeinen Formel I, in der $R_2$ eine der in den Ansprüchen 1 bis 4 erwähnten Benzimidazol-2-yl-gruppen darstellt, eine Verbindung der allgemeinen Formel

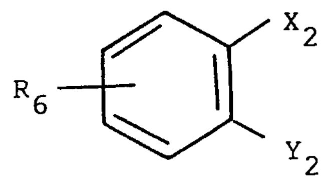

, (X)

in der
$R_6$ ein Wasserstoffatom oder in 5- oder 6-Stellung ein Fluoratom,
einer der Reste $X_2$ oder $Y_2$ eine $R_7$-NH-Gruppe und der andere der Reste $X_2$ oder $Y_2$ eine Gruppe der allgemeinen Formel

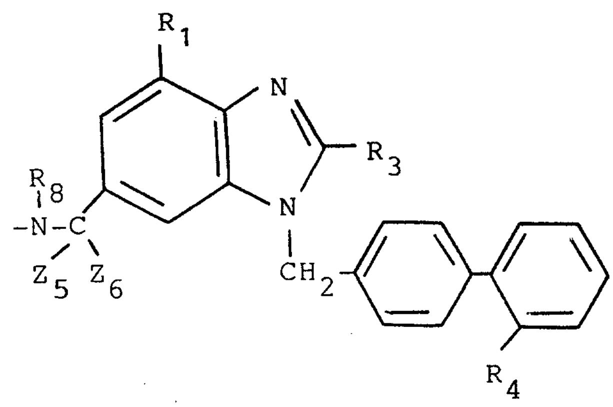

darstellen, wobei
wobei $R_1$, $R_3$ und $R_4$ wie in den Ansprüchen 1 bis 4 definiert sind,
einer der Reste $R_7$ oder $R_8$ ein Wasserstoffatom und der andere der Reste $R_7$ oder $R_8$ eine Methylgruppe,
$Z_5$ und $Z_6$, die gleich oder verschieden sein können, gegebenenfalls substituierte Aminogruppen oder gegebenenfalls durch niedere Alkylgruppen substituierte Hydroxy- oder Mercaptogruppen oder
$Z_5$ und $Z_6$, zusammen ein Sauerstoff- oder Schwefelatom,
eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten,
umgesetzt und gegebenenfalls eine so erhaltene Verbindung hydrolysiert wird und
erforderlichenfalls ein während der Umsetzungen a) bis g) zum Schutze von reaktiven Gruppen verwendeter Schutzrest abgespalten wird und/oder
gewünschtenfalls anschließend ein so erhaltenes 1-, 3-Isomerengemisch einer Verbindung der allgemeinen Formel I mittels Isomerentrennung in ihr 1- und 3-Isomer aufgetrennt wird oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.